# EUROPEAN PATENT APPLICATION

(11) **EP 2 962 656 A1**
(43) Date of publication of application: **06.01.2016**
(21) Application number: 15764693.6
(22) Date of filing: 19.03.2015
(51) Int. Cl.: A61C 5/08, A61C 13/087, A61L 27/14

(54) **TEMPORARY RESIN-BASED CROWN**

(30) Priority: 20.03.2014 KR 20140032666
(71) Applicant: Hitem Co. Ltd., Gwangju 500-480 (KR)
(72) Inventor: BACK, Hyun Young, Gwangju 502-743 (KR); SONG, Jeong Gi, Gwangju 502-743 (KR); AHN, Yong Gook, Gwangju 500-787 (KR); HAN, In Hae, Gwangju 502-755 (KR); HAN, Hyung Ryeol, Gwangju 502-755 (KR)
(74) Representative: Andrews, Robert
(86) International application number: PCT/KR2015/002698
(87) International publication number: WO 2015/142088

(57) **Abstract**

The present invention relates to a temporary resin-based crown that is used in dental prosthetic surgery to protect a prepared tooth before a final prosthesis is mounted on the prepared tooth, to a method for manufacturing the temporary resin-based crown, to a set of temporary resin-based crowns, and to a method for utilizing the set of temporary resin-based crowns. The temporary resin-based crown includes a body made of a thermoplastic resin softened at a temperature of 50 to 90°C and having a hollow portion formed at the interior thereof, the body being standardized by the occlusal surface of a tooth, the outer shape of a tooth, a crown length, a mesiodistal crown diameter, a mesiodistal cervical area diameter, and labia-lingual and buccolingual crown diameters, and one temporary resin-based crown or two or more temporary resin-based crowns connected to each other is (are) provided.

## Description

### [Technical Field]

The present invention relates to a temporary resin-based crown that is used in dental prosthetic surgery to protect a prepared tooth before a final prosthesis is mounted on the prepared tooth, to a method for manufacturing the temporary resin-based crown, to a set of temporary resin-based crowns, and to a method for utilizing the set of temporary resin-based crowns.

### [Background Art]

Generally, if teeth are damaged due to tooth decay, periodontal diseases and tooth extraction, it is hard to chew food, and further, the shape of a patient's face becomes gradually deformed asymmetrically to make him or her feel stressed when someone sees him or her. Moreover, the damaged or extracted portions of the teeth should be rapidly treated and replaced with artificial teeth so as to ensure his or her healthy living.

So as to treat the teeth damaged due to tooth decay, periodontal diseases and tooth extraction, various prosthetic surgeries like implants, bridges and false teeth have been developed. The known prosthetic surgeries need temporary prostheses during a given healing period after a damaged or extracted portion of the tooth is treated. Particularly, in case of the implant surgeries widely conducted in the prosthetic surgeries, an implant is planted in a jawbone and after that, a given healing period is passed to achieve the osseointegration of the implant into the jawbone. During the healing period, an artificial tooth is not supplemented to the permanent implant, and when the osseointegration of the permanent implant is finished after the jawbone around the planted portion is healed, the artificial tooth should be supplemented to the permanent implant. Accordingly, no stress has to be applied to the permanent implant so as to prevent the process of the osseointegration from being inhibited during the healing period, and further, a temporary tooth prosthesis is needed to prevent stress generated by the chewing operations conducted when food is taken from being applied to the permanent implant.

A temporary tooth serves to prevent the occurrence of tooth sensitivity and the movements in position of a prepared tooth before a final prosthesis is mounted on the prepared tooth. So as to make the temporary tooth, a self-curing resin is agitated and made in conformity with the prepared tooth. In this case, the temporary tooth is fitted to the prepared tooth by means of a direct method in which it is made and fitted to the prepared tooth directly by a dentist or a dental hygienist in his or her dental clinic and an indirect method in which it is made with an impression by a dental technician and fitted to the prepared tooth in the dental clinic. In case of the direct method, skilled techniques of the dentist or the dental hygienist and a lot of time for making and fitting the temporary tooth are needed. In a dental laboratory, further, the skilled techniques of the dental technician are also needed, and work time for 30 minutes to one hour is required to make one temporary tooth. Accordingly, most of dentists or dental hygienists avoid the work for making the temporary tooth.

According to the direct method, first, a patient's tooth is prepared, and after the patient sits on a treatment chair, the agitated resin is repeatedly inserted and drawn into/from his or her mouth. As the resin is hardened during the repeated insertion and drawing processes, it is heated over 50°C, and accordingly, the resin is drawn from the prepared tooth before hardened and gets cold with water so as to prevent the nerves of the prepared tooth from being damaged. In more detail, the agitated resin is located at the prepared tooth and the occlusal state of the patient is registered through a dentist's manipulation. At this time, however, the resin is spread laterally to cause the block formed by the dentist's hand to be deformed, thus making it hard to achieve one time registration. After the agitated resin is completely hardened, accordingly, it is filled again into the interior of the temporary tooth not contacted with the prepared tooth, thus allowing the internal surfaces of the prepared tooth and the temporary tooth to be brought into close contact with each other. After that, the hardened resin is removed in conformity with the shape of the tooth to form the outer shape of the temporary tooth, the height of the temporary tooth is adjusted appropriately with respect to adjacent teeth, and the occlusal relation with an antagonist tooth is finally adjusted, thus providing the temporary tooth capable of chewing food during the treatment period, without having any tooth sensitivity.

According to this method, work time for 30 minutes to one hour is required to make one temporary tooth, and therefore, most of dentists or dental hygienists avoid the work for making the temporary tooth.

### [Disclosure]

### [Technical Problem]

Accordingly, the present invention has been made in view of the above-mentioned problems occurring in the prior art, and it is an object of the present invention to provide a temporary resin-based crown and a method for manufacturing the same wherein a temporary tooth to be fitted appropriately to a prepared tooth is made by a dentist or a dental hygienist (in his or her dental clinic).

It is another object of the present invention to provide a temporary resin-based crown and a method for manufacturing the same wherein a temporary tooth to be fitted appropriately to a prepared tooth is made by a dental technician in a simple manner.

It is yet another object of the present invention to provide a temporary resin-based crown and a method for manufacturing the same wherein a temporary tooth is fitted appropriately to a prepared tooth so as to prevent the occurrence of tooth sensitivity of the prepared tooth, to avoid the movements in position of the prepared tooth, and to reduce the pain of a patient during his or her dental care.

### [Technical Solution]

To accomplish the above-mentioned objects, according to a first aspect of the present invention, there is provided a temporary resin-based crown including a body made of a thermoplastic resin softened at a temperature of 50 to 90°C and having a hollow portion formed at the interior thereof, the body being standardized by the occlusal surface of a tooth, the outer shape of a tooth, a length of a crown, a mesiodistal diameter of a crown, a mesiodistal diameter of a cervical area, and labialingual and buccolingual diameters of a crown, wherein one temporary resin-based crown or two or more temporary resin-based crowns connected to each other is (are) provided.

According to the present invention, preferably, the thermoplastic resin is a biodegradable polymer resin, and the biodegradable polymer resin includes one or two or more materials selected from the group consisting of polylactic acid (PLA), polyglycolic acid (PGA), poly-ε-caprolactone, polydioxanone, polylactic-co-glycolic acid (PLGA), polydioxanone-co-ε-caprolactone, polylactic acid-co-ε-caprolactone, polyhydroxybutyric acid-co-hydroxyvleric acid, polyphosphoester, polyethylene oxide-co-polylactic acid, polyethylene oxide-co-polylactic-co-glycolic acid, and polyethylene oxide-co-poly-ε-caprolactone.

According to the present invention, preferably, a set of temporary resin-based crowns are provided wherein the temporary resin-based crowns are standardized by any one of the following conditions:
(a) primary teeth and permanent teeth;
(b) anterior teeth, canines, premolars, and molars;
(c) one tooth, and four, six and fourteen teeth; and
(d) large, middle and small sizes determined with respect to any one of the occlusal surface of a tooth, the outer shape of a tooth, a length of a crown, a mesiodistal diameter of a crown, a mesiodistal diameter of a cervical area, and labialingual and buccolingual diameters of a crown.

To accomplish the above-mentioned objects, according to a second aspect of the present invention, there is provided a method for manufacturing a temporary resin-based crown including the steps of: preparing a temporary tooth crown mold having a tooth shape standardized with respect to the occlusal surface of a tooth, the outer shape of a tooth, a length of a crown, a mesiodistal diameter of a crown, a mesiodistal diameter of a cervical area, and labialingual and buccolingual diameters of a crown; and injecting a thermoplastic resin having properties of being softened at a temperature of 50 to 90°C into the mold, and molding the thermoplastic resin to the tooth shape.

According to the present invention, preferably, there is provided a method for manufacturing a temporary resin-based crown so as to apply the temporary resin-based crown to a patient.

To accomplish the above-mentioned objects, according to a third aspect of the present invention, there is provided a method for manufacturing a temporary resin-based crown so as to apply the temporary resin-based crown to a patient, the method including the steps of: softening the temporary resin-based crown at a temperature of 50 to 90°C; fitting the hollow portion of the softened temporary resin-based crown to a prepared tooth; deforming the body of the temporary resin-based crown fitted to the prepared tooth by means of a pressure of an antagonist tooth; hardening the body of the deformed temporary resin-based crown; separating the hardened temporary resin-based crown from the prepared tooth; and polishing the surface of the separated temporary resin-based crown.

To accomplish the above-mentioned objects, according to a fourth aspect of the present invention, there is provided a method for manufacturing a temporary resin-based crown so as to apply the temporary resin-based crown to a patient, the method including the steps of: softening the temporary resin-based crown at a temperature of 50 to 90°C; fitting the hollow portion of the softened temporary resin-based crown to a prepared tooth; deforming the body of the temporary resin-based crown fitted to the prepared tooth by means of a pressure of an antagonist tooth; hardening the body of the deformed temporary resin-based crown; separating the hardened temporary resin-based crown from the prepared tooth; filling a self-curing resin into the hollow portion of the removed temporary resin-based crown; and polishing the surface of the temporary resin-based crown filled with the self-curing resin.

To accomplish the above-mentioned objects, according to a fifth aspect of the present invention, there is provided a method for manufacturing a temporary resin-based crown so as to apply the temporary resin-based crown to a patient, the method including the steps of: softening the temporary resin-based crown at a temperature of 50 to 90°C; fitting the hollow portion of the softened temporary resin-based crown to a prepared tooth; deforming the body of the temporary resin-based crown fitted to the prepared tooth by means of a pressure of an antagonist tooth; hardening the body of the deformed temporary resin-based crown; separating the hardened temporary resin-based crown from the prepared tooth; filling a self-curing resin into the hollow portion of the separated temporary resin-based crown; polishing the surface of the temporary resin-based crown filled with the self-curing resin; and fitting the polished temporary resin-based crown to the prepared tooth and removing the self-curing resin protruding from the temporary resin-based crown.

### [Advantageous Effects]

According to the present invention, the temporary resin-based crown is made of materials not used in conventional dental treatments, easily used for prosthetic treatments by a dentist, a dental hygienist, or a dental technician, allows pain of a patient to be reduced, and permits the dental treatments to be finished in a short time.

### [Description of Drawings]

FIGS.la and 1b are perspective views showing a configuration of a temporary resin-based crown according to the present invention.
FIGS.2a and 2b are perspective views showing a configuration of a set of temporary resin-based crowns including four permanent molar teeth according to the present invention.
FIG.3 is a flow chart showing a method for manufacturing the temporary resin-based crown according to the present invention.
FIGS.4a to 4h are photographs showing the method for manufacturing the temporary resin-based crown for one molar according to the present invention.
FIGS.5a to 5h are photographs showing the method for manufacturing the set of temporary resin-based crowns for six anterior teeth according to the present invention.

### [Mode for Invention]

The present invention relates to a temporary resin-based crown that includes a body made of a thermoplastic resin softened at a temperature of 50 to 90°C and having a hollow portion formed at the interior thereof, wherein the body is standardized by the occlusal surface of a tooth, the outer shape of a tooth, a length of a crown, a mesiodistal diameter of a crown, a mesiodistal diameter of a cervical area, and labialingual and buccolingual diameters of a crown.

A self-curing resin used for conventional temporary resin-based crown is polymerized and cured at a normal temperature in a short time, but heat is generated from the self-curing resin during the curing to cause the teeth and nerves around a portion at which a prosthetic surgery is conducted to be damaged, so that there is a need to pay considerable attention to the usage of the self-curing resin. To the contrary, the thermoplastic resin used in the present invention is softened at a temperature of 50 to 90°C and cured at a normal temperature in a short time, without having the heat generated therefrom.

According to the present invention, desirably, the thermoplastic resin is a biodegradable polymer resin. The biodegradable polymer resin includes one or two or more materials selected from the group consisting of polylactic acid (PLA), polyglycolic acid (PGA), poly-ε-caprolactone, polydioxanone, polylactic-co-glycolic acid (PLGA), polydioxanone-co-ε-caprolactone, polylactic acid-co-ε-caprolactone, polyhydroxybutyric acid-co-hydroxyvleric acid, polyphosphoester, polyethylene oxide-co-polylactic acid, polyethylene oxide-co-polylactic-co-glycolic acid, and polyethylene oxide-co-poly-ε-caprolactone. Desirably, the biodegradable polymer resin includes one or more materials selected from the group consisting of polylactic acid (PLA), polyglycolic acid (PGA), poly-ε-caprolactone, and polylactic-co-glycolic acid (PLGA), and more desirably, it includes polylactic acid (PLA).

According to the present invention, a temporary resin-based crown 10 includes a body 100 and a hollow portion 200. The body 100 is standardized by the occlusal surface of a tooth, the outer shape of a tooth, a length of a crown, a mesiodistal diameter of a crown, a mesiodistal diameter of a cervical area, and labialingual and buccolingual diameters of a crown. Further, the hollow portion 200 is formed at the interior of the body 100 in such a manner as to be open at the top portion of the body 100.

The hollow portion 200 is a space to which a prepared tooth is fitted, and if the hollow portion 200 has a spare space therein after the prepared tooth is fitted, the spare space is filled with a self-curing resin. In case where the temporary resin-based crown 10 according to the present invention is applied not to the prepared tooth, but to a portion from which a tooth is extracted, the hollow portion 200 is filled with the self-curing resin. Further, if the number of missing teeth is two or more and the left and right teeth with respect to the missing teeth are prepared, the hollow portions 200 for the missing teeth are filled with the self-curing resin, and the prepared teeth are fitted to the hollow portions 200 filled or not filled with the self-curing resin.

According to the present invention, the prepared tooth means a tooth whose enamel is partially or entirely polished, as an object of a prosthetic treatment restoring damages thereon due to tooth decay, periodontal diseases, and tooth extraction.

According to the present invention, the standards of the body of the crown are determined by size in accordance with the occlusal surface of a tooth, the outer shape of a tooth, a length of a crown, a mesiodistal diameter of a crown, a mesiodistal diameter of a cervical area, and labialingual and buccolingual diameters of a crown.

For example, permanent teeth constituting upper and lower jaws are classified into large, middle and small-sized teeth in accordance with the occlusal surface of a tooth, the outer shape of a tooth, a length of a crown, a mesiodistal diameter of a crown, a mesiodistal diameter of a cervical area, and labialingual and buccolingual diameters of a crown. In more detail, Table 1 indicates the large-sized teeth, Table 2 the middle-sized teeth, and Table 3 the small-sized teeth. Primary teeth have a single size for the occlusal surface of a tooth, the outer shape of a tooth, a length of a crown, a mesiodistal diameter of a crown, a mesiodistal diameter of a cervical area, and labialingual and buccolingual diameters of a crown, respectively. Table 4 indicates the standards of the primary teeth. Tables 1 to 3 suggest the standards of the permanent teeth, and Table 4 the standards of the primary teeth. However, the standards are not necessarily limited to those suggested below.

**Table 1**

| Permanent teeth (unit=mm) | | Crown length | Mesiodistal crown diameter | Mesiodistal cervical area diameter | Labialingual and buccolingual crown diameter |
|---|---|---|---|---|---|
| | Central incisor | 11.5 | 9.5 | 8.0 | 8.0 |
| | Lateral incisor | 10.0 | 7.5 | 6.0 | 7.0 |
| | Canine | 11.0 | 8.5 | 6.5 | 9.0 |
| Upper jaw | First premolar | 9.5 | 8.0 | 6.0 | 10.0 |
| | Second premolar | 9.5 | 8.0 | 6.0 | 10.0 |
| | First molar | 8.5 | 11.0 | 9.0 | 12.0 |
| | Second molar | 8.0 | 10.0 | 8.0 | 12.0 |
| | Central incisor | 10.0 | 6.0 | 4.5 | 7.0 |
| | Lateral incisor | 10.5 | 6.5 | 5.0 | 7.5 |
| Lower jaw | Canine | 12.0 | 8.0 | 6.5 | 8.5 |
| | First premolar | 9.5 | 8.0 | 6.0 | 8.5 |
| | Second premolar | 9.0 | 8.0 | 6.0 | 9.0 |
| | First molar | 8.5 | 12.0 | 10.0 | 11.5 |
| | Second molar | 8.0 | 11.5 | 9.0 | 11.0 |

**Table 2**

| Permanent teeth (unit=mm) | | Crown length | Mesiodistal crown diameter | Mesiodistal cervical area diameter | Labialingual and buccolingual crown diameter |
|---|---|---|---|---|---|
| | Central incisor | 10.5 | 8.5 | 7.0 | 7.0 |
| | Lateral incisor | 9.0 | 6.5 | 5.0 | 6.0 |
| | Canine | 10.0 | 7.5 | 5.5 | 8.0 |
| Upper jaw | First premolar | 8.5 | 7.0 | 5.0 | 9.0 |
| | Second premolar | 8.5 | 7.0 | 5.0 | 9.0 |
| | First molar | 7.5 | 10.0 | 8.0 | 11.0 |
| | Second molar | 7.0 | 9.0 | 7.0 | 11.0 |
| | Central incisor | 9.0 | 5.0 | 3.5 | 6.0 |
| | Lateral incisor | 9.5 | 5.5 | 4.0 | 6.5 |
| | Canine | 11.0 | 7.0 | 5.5 | 7.5 |
| Lower jaw | First premolar | 8.5 | 7.0 | 5.0 | 7.5 |
| | Second premolar | 8.0 | 7.0 | 5.0 | 8.0 |
| | First molar | 7.5 | 11.0 | 9.0 | 10.5 |
| | Second molar | 7.0 | 10.5 | 8.0 | 10.0 |

**Table 3**

| Permanent teeth (unit=mm) | | Crown length | Mesiodistal crown diameter | Mesiodistal cervical area diameter | Labialingual and buccolingual crown diameter |
|---|---|---|---|---|---|
| | Central incisor | 9.5 | 7.5 | 6.0 | 6.0 |
| | Lateral incisor | 8.0 | 5.5 | 4.0 | 5.0 |
| | Canine | 9.0 | 6.5 | 4.5 | 7.0 |
| Upper jaw | First premolar | 7.5 | 6.0 | 4.0 | 8.0 |
| | Second premolar | 7.5 | 6.0 | 4.0 | 8.0 |
| | First molar | 6.5 | 9.0 | 7.0 | 10.0 |
| | Second molar | 6.0 | 8.0 | 6.0 | 10.0 |
| | Central incisor | 8.0 | 4.0 | 2.5 | 5.0 |
| | Lateral incisor | 8.5 | 4.5 | 3.0 | 5.5 |
| | Canine | 10.0 | 6.0 | 4.5 | 6.5 |
| Lower jaw | First premolar | 7.5 | 6.0 | 4.0 | 6.5 |
| | Second premolar | 7.0 | 6.0 | 4.0 | 7.0 |
| | First molar | 6.5 | 10.0 | 8.0 | 9.5 |
| | Second molar | 6.0 | 9.5 | 7.0 | 9.0 |

**Table 4**

| Primary teeth (unit=mm) | | Crown length | Mesiodistal crown diameter | Mesiodistal cervical area diameter | Labialingual and buccolingual crown diameter |
|---|---|---|---|---|---|
| | Central incisor | 6.0 | 6.5 | 4.5 | 5.0 |
| Upper jaw | Lateral incisor | 5.6 | 5.1 | 3.7 | 4.8 |
| | Canine | 6.5 | 7.0 | 5.1 | 7.0 |
| | Primary first molar | 5.1 | 7.3 | 5.2 | 8.5 |
| | Primary second molar | 5.7 | 8.2 | 6.4 | 10.0 |
| | Central incisor | 5.0 | 4.2 | 3.0 | 4.0 |
| Lower jaw | Lateral incisor | 5.2 | 4.1 | 3.0 | 4.0 |
| | Canine | 6.5 | 5.0 | 3.7 | 4.8 |
| | Primary first molar | 6.0 | 7.7 | 6.5 | 7.0 |
| | Primary second molar | 5.5 | 9.9 | 7.2 | 8.7 |

As listed above, the temporary resin-based crowns for the permanent teeth are made by the standards indicated in Tables 1 to 3, and the temporary resin-based crowns for the primary teeth are made by the standards indicated in Table 4.

The present invention relates to a single temporary resin-based crown, and further, to two or more temporary resin-based crowns connected to each other.

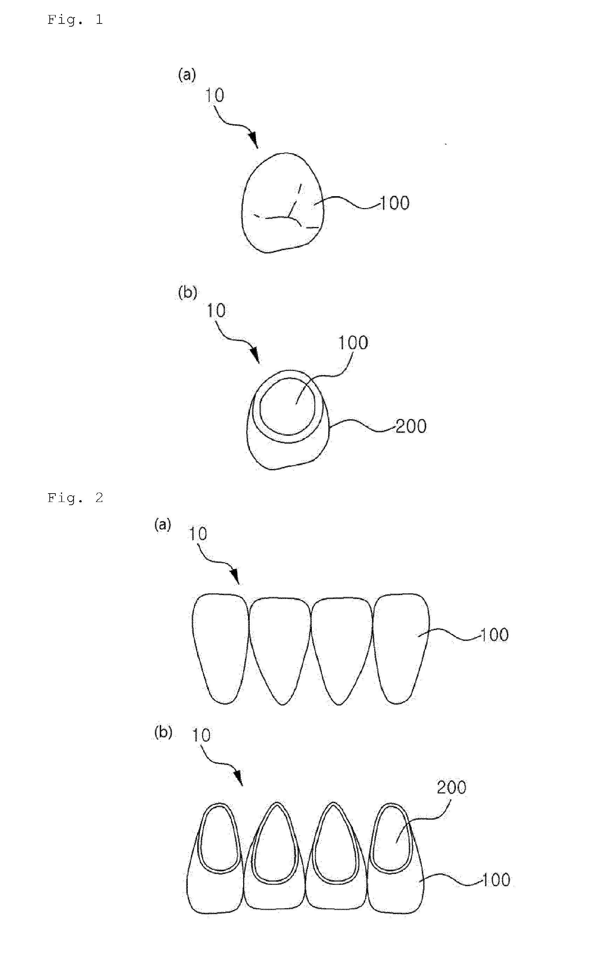

For example, FIGS.2a and 2b are perspective views showing a configuration of a set of temporary resin-based crowns including four permanent molar teeth according to the present invention.

In addition to one prepared tooth, as shown, the temporary resin-based crown according to the present invention may be applied to two or more prepared teeth or to two or more prepared teeth and one or more extracted teeth. At this time, the connection of the teeth means the bodies of the temporary resin-based crowns are connected to each other.

Further, the present invention provides a set of temporary resin-based crowns including a plurality of standardized temporary resin-based crowns.

In more detail, the set of temporary resin-based crowns includes the temporary resin-based crowns for primary teeth and permanent teeth.

Further, the set of temporary resin-based crowns includes the temporary resin-based crowns for anterior teeth, canines, premolars, and molars.

Furthermore, the set of temporary resin-based crowns includes the temporary resin-based crowns for a tooth, four teeth, six teeth, and fourteen teeth.

Also, the set of temporary resin-based crowns is classified into large, middle and small-sized sets of temporary resin-based crowns that are determined with respect to any one of the occlusal surface of a tooth, the outer shape of a tooth, a length of a crown, a mesiodistal diameter of a crown, a mesiodistal diameter of a cervical area, and labialingual and buccolingual diameters of a crown.

Next, a method for manufacturing the temporary resin-based crown according to the present invention includes the steps of: preparing a temporary tooth crown mold having a tooth shape standardized with respect to the occlusal surface of a tooth, the outer shape of a tooth, a length of a crown, a mesiodistal diameter of a crown, a mesiodistal diameter of a cervical area, and labialingual and buccolingual diameters of a crown; and injecting a thermoplastic resin having properties of being softened at a temperature of 50 to 90°C into the mold, and molding the thermoplastic resin to the tooth shape.

According to the present invention, the mold has the standardized tooth shape, and the standards are discriminated, set and quantificated with respect to the occlusal surface of a tooth, the outer shape of a tooth, a length of a crown, a mesiodistal diameter of a crown, a mesiodistal diameter of a cervical area, and labialingual and buccolingual diameters of a crown. The standards have been already explained above.

Accordingly, a plurality of molds is prepared with respect to the standardized tooth shape, and the plurality of molds may constitute one set.

As mentioned above, the thermoplastic resin is softened at a temperature of 50 to 90°C and cured at a normal temperature in a short time, without having the heat generated therefrom. According to the present invention, desirably, the thermoplastic resin is a biodegradable polymer resin. For example, the biodegradable polymer resin includes one or two or more materials selected from the group consisting of polylactic acid (PLA), polyglycolic acid (PGA), poly-ε-caprolactone, polydioxanone, polylactic-co-glycolic acid (PLGA), polydioxanone-co-ε-caprolactone, polylactic acid-co-ε-caprolactone, polyhydroxybutyric acid-co-hydroxyvleric acid, polyphosphoester, polyethylene oxide-co-polylactic acid, polyethylene oxide-co-polylactic-co-glycolic acid, and polyethylene oxide-co-poly-ε-caprolactone. Desirably, the biodegradable polymer resin includes one or more materials selected from the group consisting of polylactic acid (PLA), polyglycolic acid (PGA), poly-ε-caprolactone, and polylactic-co-glycolic acid (PLGA), and more desirably, it includes polylactic acid (PLA).

On the other hand, the present invention relates to the method for manufacturing the temporary resin-based crown, through which the temporary resin-based crown is applied to a prosthetic treatment so as to restore a tooth damaged due to tooth decay, periodontal diseases, and tooth extraction.

In more detail, the method for manufacturing the temporary resin-based crown according to the present invention includes the steps of: softening the temporary resin-based crown at a temperature of 50 to 90°C (step S100); fitting the hollow portion of the softened temporary resin-based crown to a prepared tooth (step S200); deforming the body of the temporary resin-based crown fitted to the prepared tooth by a pressure of an antagonist tooth (step S300); hardening the body of the deformed temporary resin-based crown (step S400); separating the hardened temporary resin-based crown from the prepared tooth (step S500); and polishing the surface of the separated temporary resin-based crown (step S600).

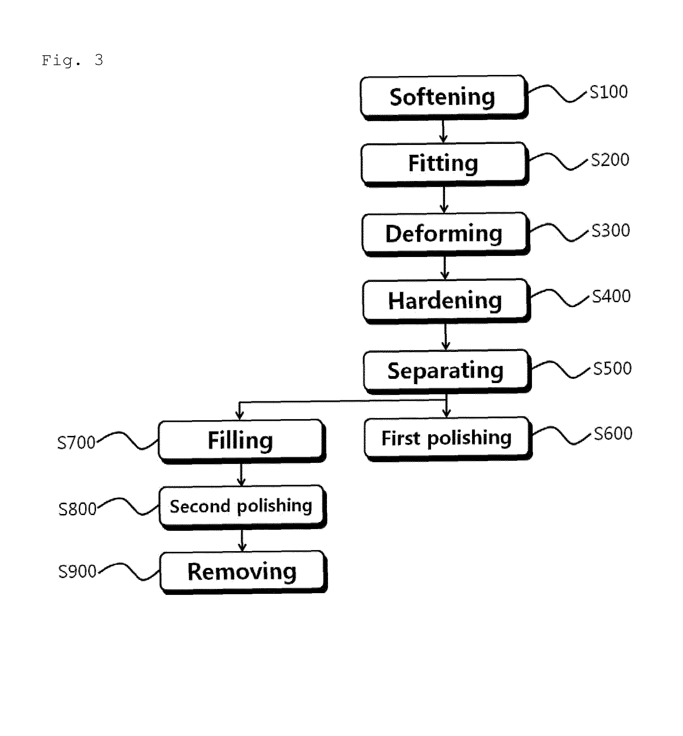

Hereinafter, the method for manufacturing the temporary resin-based crown according to the present invention will be in detail explained with reference to FIG.3.

In the softening step (S100), the temporary resin-based crown is softened at a temperature of 50 to 90°C. The softening of the temporary resin-based crown is conducted to a degree at which the temporary resin-based crown is freely deformed in the fitting step (S200) and the deforming step (S300), and desirably, the softening is conducted through the immersion of the temporary resin-based crown in water having a temperature of 50 to 90°C for 10 to 30 seconds or through the heating of the temporary resin-based crown.

In more detail, since the temporary resin-based crown according to the present invention has a standardized tooth shape, the temporary resin-based crown having the most similar standard to the prepared tooth of the patient is selected and immersed in water having a temperature of 50 to 90°C for 10 to 30 seconds or heated to the temperature, thus finishing the softening thereof.

In the fitting step (S200), the hollow portion of the temporary resin-based crown softened in the softening step (S100) is fitted to the prepared tooth.

In more detail, since the temporary resin-based crown according to the present invention has a standardized tooth shape, the temporary resin-based crown having the most similar standard to the prepared tooth of the patient is selected and then fitted to the prepared tooth of the patient.

In this case, the prepared tooth includes a tooth whose enamel is partially or entirely polished, as an object for a prosthetic treatment restoring the tooth damaged due to tooth decay, periodontal diseases, and tooth extraction.

In the deforming step (S300), the body of the temporary resin-based crown fitted to the prepared tooth in the fitting step (S200) is deformed by a pressure of an antagonist tooth.

In more detail, the body of the temporary resin-based crown fitted to the prepared tooth of the patient is deformed fittably to the outer shape of the prepared tooth and the occlusal surface thereof by means of a pressure of an antagonist tooth when the patient clamps his or her lips.

In this case, the deformation means the body of the temporary resin-based crown becomes deformed correspondingly to the occlusal surface of the prepared tooth of the patient as well as to the external shape of the prepared tooth thereof.

In the hardening step (S400), the body of the temporary resin-based crown deformed in the deforming step (S300) is hardened.

In more detail, the body of the temporary resin-based crown deformed in the deforming step (S300) correspondingly to the occlusal surface of the prepared tooth of the patient and the external shape of the prepared tooth thereof is kept hardened for a given period of time, thus allowing the outer shape of the body thereof to be just maintained.

According to the present invention, the hardening means the body of the temporary resin-based crown becomes hardened, while having given hardness. The thermoplastic resin used in the present invention is naturally hardened as it gets cold, but so as to shorten the hardening time, desirably, air being in a normal temperature is injected into the body of the temporary resin-based crown. Otherwise, water sprays to the body of the temporary resin-based crown. As a result, the hardening step is finished.

In the separating step (S500), the temporary resin-based crown hardened in the hardening step (S400) is separated from the prepared tooth.

In the polishing step (S600), the surface of the temporary resin-based crown separated from the prepared tooth (S500) is polished.

In more detail, the temporary resin-based crown hardened in the hardening step (S400) may be used directly, without being separated from the prepared tooth. Through the deformation of the body of the temporary resin-based crown in the deforming step (S300), however, the body of the temporary resin-based crown may be hardened in the hardening step (S400), while being not gentle on the outer shape thereof. In this case, desirably, the temporary resin-based crown hardened in the hardening step (S400) is separated from the prepared tooth and the surface thereof has to be polished to improve the psychological beauty of the tooth of the patient.

The polishing is conducted by using a polishing tool used generally in dental treatments.

On the other hand, in some cases, the temporary resin-based crown after the deforming step (S300) and the hardening step (S400) does not correspond to the occlusal surface of the prepared tooth of the patient and the external shape of the prepared tooth thereof, and it may have a space from the prepared tooth.

Accordingly, the method for manufacturing the temporary resin-based crown according to the present invention further includes the steps of: filling a self-curing resin into the hollow portion of the temporary resin-based crown separated in the separating step (S500) from the prepared tooth (step S700); and polishing the surface of the temporary resin-based crown filled with the self-curing resin (step S800).

In the filling step (S700), the amount of self-curing resin filled is sufficient enough to fill the space between the prepared tooth and the temporary resin-based crown when the temporary resin-based crown after the deforming step (S300) and the hardening step (S400) does not correspond to the occlusal surface of the prepared tooth of the patient and the external shape of the prepared tooth thereof.

Further, the method for manufacturing the temporary resin-based crown according to the present invention further includes the step of: fitting the temporary resin-based crown to the prepared tooth after the polishing step (S800); and removing the self-curing resin protruding from the temporary resin-based crown (step S900).

In the removing step (S900), the removal of the self-curing resin is conducted by using a spatula used generally in dental treatments.

As mentioned above, the temporary resin-based crown according to the present invention can be made by a dentist or a dental hygienist in his or her dental clinic, and further, the temporary resin-based crown to be fitted appropriately to a prepared tooth of a patient can be made by a dental technician in a simple manner. Furthermore, the temporary resin-based crown according to the present invention can be fitted appropriately to a prepared tooth so as to prevent the occurrence of tooth sensitivity of the prepared tooth, to avoid the movements in position of the prepared tooth, and to reduce the pain of the patient during his or her dental care.

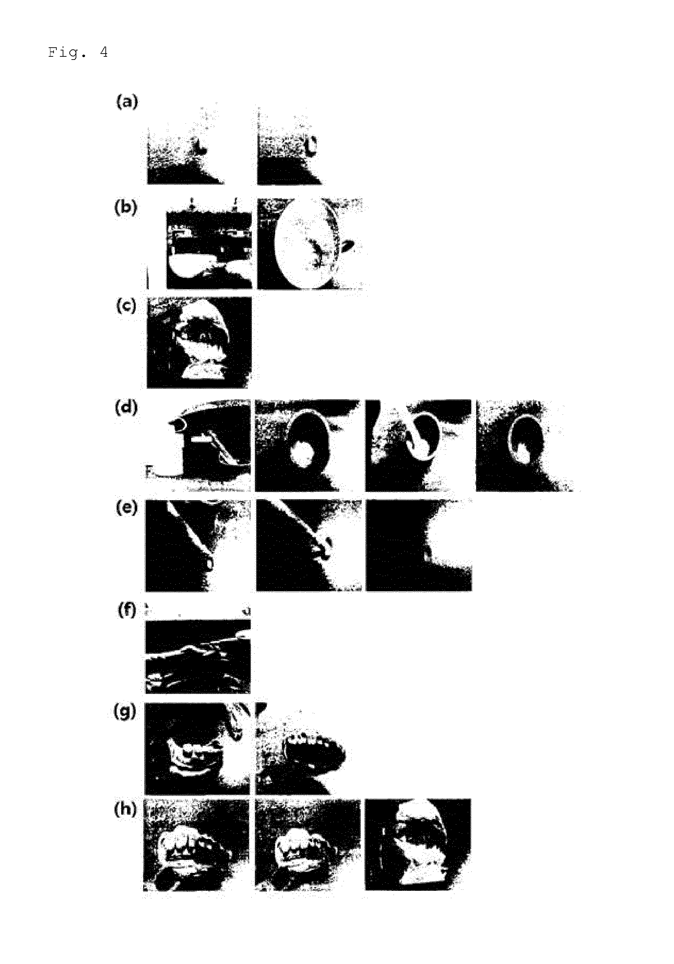

FIGS.4a to 4h show the method for manufacturing a temporary resin-based crown according to the present invention, wherein the temporary resin-based crown for one molar is fitted to a prepared tooth among the molars of a patient.

In more detail, FIG.4a is a photograph showing the temporary resin-based crown for one molar. FIG.4b is a photograph showing the state wherein the temporary resin-based crown for one molar is immersed in hot purified water (at a temperature of about 60 to 80°C) for about 20 seconds, thus allowing the temporary resin-based crown for one molar to be softened. FIG.4c is a photograph showing the state wherein the temporary resin-based crown for one molar is fitted to the prepared tooth among the patient's molars, and if it is pressed by an antagonist tooth, the softened temporary resin-based crown is pressed correspondingly to the antagonist tooth to form the occlusal surface thereon. After that, air is injected into the temporary resin-based crown or water is sprayed thereinto, and the occlusal surface of the temporary resin-based crown pressedly formed by the antagonist tooth is just hardened. FIG.4d is a photograph showing the state wherein a self-curing resin is made. FIG.4e is a photograph showing the state wherein the self-curing resin is filled into the hollow portion of the temporary resin-based crown hardened in FIG.4c. FIG.4f is a photograph showing the state wherein the body of the temporary resin-based crown longer than abutment teeth is polished correspondingly to the abutment teeth. FIG.4g is a photograph showing the state wherein the temporary resin-based crown after polished is fitted again to the prepared tooth and occluded with the antagonist tooth to allow the self-curing resin filled into the hollow portion of the temporary resin-based crown to be filled into the space formed between the temporary resin-based crown and the prepared tooth. After that, the filled self-curing resin is hardened, and the spare resin is removed from the temporary resin-based crown. FIG.4h is a photograph showing the temporary resin-based crown made finally through the above steps.

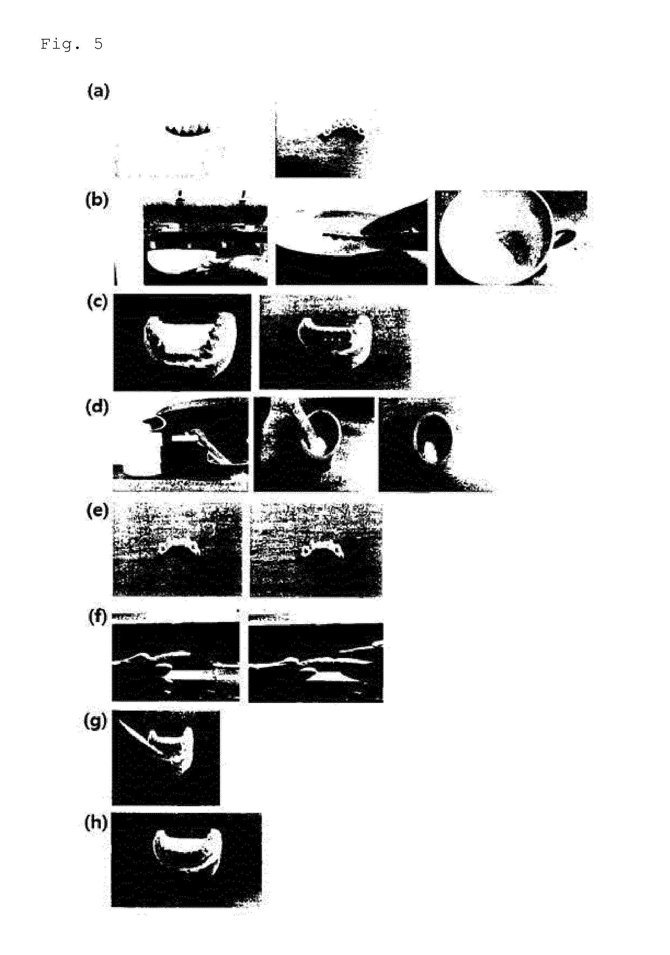

FIGS.5a to 5h show the method for manufacturing a temporary resin-based crown according to the present invention, wherein the temporary resin-based crowns for six anterior teeth connected to each other are fitted to the missing and prepared teeth of a patient.

In more detail, FIG.5a is a photograph showing the temporary resin-based crowns for six anterior teeth are connected to each other. FIG.5b is a photograph showing the state wherein the temporary resin-based crowns for six anterior teeth are immersed in hot purified water (at a temperature of about 60 to 80°C) for about 20 seconds, thus allowing the temporary resin-based crowns for six anterior teeth to be softened. FIG.5c is a photograph showing the state wherein the softened temporary resin-based crowns are fitted to the anterior region of the lower jaw, and if they are pressed by antagonist teeth, the softened temporary resin-based crowns are pressed correspondingly to the antagonist teeth to form the occlusal surfaces thereon. After that, air is injected into the temporary resin-based crowns or water is sprayed thereinto, and the occlusal surfaces of the temporary resin-based crowns pressedly formed by the antagonist teeth are just hardened. FIG.5d is a photograph showing the state wherein a self-curing resin is made. FIG.5e is a photograph showing the state wherein the self-curing resin is filled into the hollow portions of the temporary resin-based crowns hardened in FIG.5c. FIG.5f is a photograph showing the state wherein the bodies of the temporary resin-based crowns longer than abutment teeth are polished correspondingly to the prepared teeth. FIG.5g is a photograph showing the state wherein the temporary resin-based crowns after polished are fitted again to the prepared teeth and occluded with the antagonist teeth to allow the self-curing resin filled into the hollow portions of the temporary resin-based crowns to be filled into the spaces formed between the temporary resin-based crowns and the prepared teeth. After that, the filled self-curing resin is hardened, and the spare resin is removed from each temporary resin-based crowns. FIG.5h is a photograph showing the temporary resin-based crowns made finally through the above steps.

## Claims

1. A temporary resin-based crown comprising a body made of a thermoplastic resin softened at a temperature of 50 to 90°C and having a hollow portion formed at the interior thereof, the body being standardized by the occlusal surface of a tooth, the outer shape of a tooth, a crown length, a mesiodistal crown diameter, a mesiodistal cervical area diameter, and labia-lingual and buccolingual crown diameters, wherein one temporary resin-based crown or two or more temporary resin-based crowns connected to each other is (are) provided.

2. The temporary resin-based crown according to claim 1, wherein the thermoplastic resin is a biodegradable polymer resin.

3. The temporary resin-based crown according to claim 2, wherein the biodegradable polymer resin comprises one or two or more materials selected from the group consisting of polylactic acid (PLA), polyglycolic acid (PGA), poly-ε-caprolactone, polydioxanone, polylactic-co-glycolic acid (PLGA), polydioxanone-co-ε-caprolactone, polylactic acid-co-ε-caprolactone, polyhydroxybutyric acid-co-hydroxyvleric acid, polyphosphoester, polyethylene oxide-co-polylactic acid, polyethylene oxide-co-polylactic-co-glycolic acid, and polyethylene oxide-co-poly-ε-caprolactone.

4. A set of temporary resin-based crowns according to any one of claims 1 to 3, wherein the temporary resin-based crowns are standardized by any one of the following conditions:
(e) primary teeth and permanent teeth;
(f) anterior teeth, canines, premolars, and molars;
(g) one tooth, and four, six and fourteen teeth; and
(h) large, middle and small sizes determined with respect to any one of the occlusal surface of a tooth, the outer shape of a tooth, a length of a crown, a mesiodistal diameter of a crown, a mesiodistal diameter of a cervical area, and labialingual and buccolingual diameters of a crown.

5. A method for manufacturing a temporary resin-based crown according to any one of claims 1 to 3, the method comprising the steps of:
softening the temporary resin-based crown at a temperature of 50 to 90°C;
fitting the hollow portion of the softened temporary resin-based crown to a prepared tooth;
deforming the body of the temporary resin-based crown fitted to the prepared tooth by means of a pressure of an antagonist tooth;
hardening the body of the deformed temporary resin-based crown;
separating the hardened temporary resin-based crown from the prepared tooth; and
polishing the surface of the separated temporary resin-based crown.

6. A method for manufacturing a temporary resin-based crown according to any one of claims 1 to 3, the method comprising the steps of:
softening the temporary resin-based crown at a temperature of 50 to 90°C;
fitting the hollow portion of the softened temporary resin-based crown to a prepared tooth;
deforming the body of the temporary resin-based crown fitted to the prepared tooth by means of a pressure of an antagonist tooth;
hardening the body of the deformed temporary resin-based crown;
separating the hardened temporary resin-based crown from the prepared tooth;
filling a self-curing resin into the hollow portion of the separated temporary resin-based crown; and
polishing the surface of the temporary resin-based crown filled with the self-curing resin.

7. A method for manufacturing a temporary resin-based crown according to any one of claims 1 to 3, the method comprising the steps of:
softening the temporary resin-based crown at a temperature of 50 to 90°C;
fitting the hollow portion of the softened temporary resin-based crown to a prepared tooth;
deforming the body of the temporary resin-based crown fitted to the prepared tooth by means of a pressure of an antagonist tooth;
hardening the body of the deformed temporary resin-based crown;
separating the hardened temporary resin-based crown from the prepared tooth;
filling a self-curing resin into the hollow portion of the separated temporary resin-based crown;
polishing the surface of the temporary resin-based crown filled with the self-curing resin; and
fitting the polished temporary resin-based crown to the prepared tooth and removing the self-curing resin protruding from the temporary resin-based crown.
